(19) Europäisches Patentamt
European Patent Office
Office européen des brevets

(11) Publication number: 0 398 998 B1

(12) **EUROPEAN PATENT SPECIFICATION**

(45) Date of publication of patent specification: **05.01.94**

(51) Int. Cl.[5]: **C01B 33/34**, C01B 35/12

(21) Application number: **89905023.1**

(22) Date of filing: **28.11.88**

(86) International application number:
**PCT/US88/04251**

(87) International publication number:
**WO 90/06283 (14.06.90 90/14)**

(54) **SYNTHETIC POROUS CRYSTALLINE MATERIAL, ITS SYNTHESIS AND USE.**

(43) Date of publication of application:
**28.11.90 Bulletin 90/48**

(45) Publication of the grant of the patent:
**05.01.94 Bulletin 94/01**

(84) Designated Contracting States:
**BE DE FR GB IT NL SE**

(56) References cited:
**EP-A- 0 293 032**
**US-A- 4 439 409**
**US-A- 4 442 222**
**US-A- 4 537 754**
**US-A- 4 678 766**

(73) Proprietor: **MOBIL OIL CORPORATION**
**150 East 42nd Street**
**New York New York 10017(US)**

(72) Inventor: **CHU, Pochen**
**1 Cranberry Place**
**Voorhees, New Jersey 08043(US)**
Inventor: **RUBIN, Mae, Koenig**
**50 Belmont Avenue**
**Bala Cynwyd, PA 19004(US)**

(74) Representative: **Colmer, Stephen Gary et al**
**Patent Department**
**Mobil Court**
**3 Clements Inn**
**London WC2A 2EB (GB)**

EP 0 398 998 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a synthetic porous crystalline material, to a method for its preparation and to its use in catalytic conversion of organic compounds.

Zeolitic materials, both natural and synthetic, have been demonstrated in the past to have catalytic properties for various types of hydrocarbon conversion. Certain zeolitic materials are ordered, porous crystalline aluminosilicates having a definite crystalline structure as determined by X-ray diffraction, within which there are a large number of smaller cavities which may be interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties.

Such molecular sieves, both natural and synthetic, include a wide variety of positive ion-containing crystalline silicates. These silicates can be described as a rigid three-dimensional framework of $SiO_4$ and Periodic Table Group IIIA element oxide, e.g. $AlO_4$, in which the tetrahedra are cross-linked by the sharing of oxygen atoms whereby the ratio of the total Group IIIA element, e.g. aluminum, and silicon atoms to oxygen atoms is 1:2. The electrovalence of the tetrahedra containing the Group IIIA element, e.g. aluminum, is balanced by the inclusion in the crystal of a cation, for example an alkali metal or an alkaline earth metal cation. This can be expressed wherein the ratio of the Group IIIA element, e.g. aluminum, to the number of various cations, such as Ca/2, Sr/2, Na, K or Li, is equal to unity. One type of cation may be exchanged either entirely or partially with another type of cation utilizing ion exchange techniques in a conventional manner. By means of such cation exchange, it has been possible to vary the properties of a given silicate by suitable selection of the cation.

Prior art techniques have resulted in the formation of a great variety of synthetic zeolites. Many of these zeolites have come to be designated by letter or other convenient symbols, as illustrated by zeolite A (U.S. Patent 2,882,243), zeolite X (U.S. Patent 2,882,244), zeolite Y (U.S. Patent 3,130,007), zeolite ZK-5 (U.S. Patent 3,247,195), zeolite ZK-4 (U.S. Patent 3,314,752), zeolite ZSM-5 (U.S. Patent 3,702,886), zeolite ZSM-11 (U.S. Patent 3,709,979), zeolite ZSM-12 (U.S. Patent 3,832,449), zeolite ZSM-20 (U.S. Patent 3,972,983), ZSM-35 (U.S. Patent 4,016,245) and zeolite ZSM-23 (U.S. Patent 4,076,842).

The $SiO_2/Al_2O_3$ ratio of a given zeolite is often variable. For example, zeolite X can be synthesized with $SiO_2/Al_2O_3$ ratios of from 2 to 3; zeolite Y, from 3 to 6. In some zeolites, the upper limit of the $SiO_2/Al_2O_3$ ratio is unbounded. ZSM-5 is one such example wherein the $SiO_2/Al_2O_3$ ratio is at least 5 and up to the limits of present analytical measurement techniques. U.S. Patent 3,941,871 (Re. 29,948) discloses a porous crystalline silicate made from a reaction mixture containing no deliberately added alumina and exhibiting the X-ray diffraction pattern characteristic of ZSM-5. U.S. Patents 4,061,724, 4,073,865 and 4,104,294 describe crystalline silicate of varying alumina and metal content.

In a first aspect, the invention resides in a synthetic porous crystalline material characterized by an X-ray diffraction pattern including values substantially as set forth in Table I of the specification and having equilibrium adsorption capacities of greater than 10 wt.% for water vapor, greater than 4.5 wt.% for cyclohexane vapor and greater than 10 wt.% for n-hexane vapor.

The porous crystalline material of the invention appears to be related to the compositions named "PSH-3" described in U.S. Patent 4,439,409. However, the instant crystalline material does not appear to contain all the components apparently present in the PSH-3 compositions. In particular, the composition of this invention is not contaminated with other crystal structures, such as ZSM-12 or ZSM-5, exhibits unusual sorption capacities and unique catalytic utility when compared to the PSH-3 compositions synthesized in accordance with U.S Patent 4,439,409.

The crystalline material of the invention can be synthesized by crystallization of a reaction mixture containing sources of the required oxides together with a hexamethyleneimine directing agent. It is, however, found particularly where the crystalline material of the invention is a silicate, that it is important to use a silica source with a relatively high solids content.

Accordingly, in a second aspect the invention resides in a method for preparing the synthetic crystalline material according to said one aspect of the invention comprising preparing a reaction mixture capable of forming said material upon crystallization, said reaction mixture containing sufficient amounts of alkali or alkaline earth metal cations, a source of tetravalent oxide $YO_2$ containing at least about 30 wt.% solid $YO_2$, a source of trivalent oxide $X_2O_3$, water and hexamethyleneimine, and maintaining said reaction mixture under sufficient crystallization conditions until crystals of said material are formed.

The crystalline material of this invention has a composition involving the molar relationship:

$X_2O_3:(n)YO_2$,

wherein X is a trivalent element, such as aluminum, boron, iron and/or gallium, preferably aluminum, Y is a tetravalent element such as silicon and/or germanium, preferably silicon, and n is at least 10, usually from 10 to 150, preferably from 10 to 60, and most preferably from 20 to 40. In the as-synthesized form, the material has a formula, on an anhydrous basis and in terms of moles of oxides per n moles of $YO_2$, as follows:

$(0.005\text{-}0.1)Na_2O:(1\text{-}4)R:X_2O_3:nYO_2$

wherein R is an organic moiety. The Na and R components are associated with the material as a result of their presence during crystallization, and are easily removed by post-crystallization methods hereinafter more particularly described.

The crystalline material of the invention is thermally stable and exhibits high surface area (greater than 400 $m^2$/gm) and unusually large sorption capacity when compared to similar crystal structures. As is evident from the above formula, the crystalline material of this invention is synthesized nearly free of Na cations. It can, therefore, be used as a catalyst with acid activity without an exchange step. To the extent desired, however, the original sodium cations of the as-synthesized material can be replaced in accordance with techniques well known in the art, at least in part, by ion exchange with other cations. Preferred replacing cations include metal ions, hydrogen ions, hydrogen precursor, e.g. ammonium, ions and mixtures thereof. Particularly preferred cations are those which tailor the catalytic activity for certain hydrocarbon conversion reactions. These include hydrogen, rare earth metals and metals of Groups IIA, IIIA, IVA, IB, IIB, IIIB, IVB and VIII of the Periodic Table of the Elements.

In its calcined form, the crystalline material of the invention appears to be made up of a single crystal phase with little or no detectable impurity crystal phases and has an X-ray diffraction pattern which is distinguished from the patterns of other known crystalline materials by the lines listed in Table I below:

TABLE I

| Interplanar d-Spacing (A) | Relative Intensity, I/Io x 100 |
|---|---|
| 30.0 ± 2.2 | W-M |
| 22.1 ± 1.3 | W |

more specifically by the lines listed in Table II below:

TABLE II

| Interplanar d-Spacing (A) | Relative Intensity, I/Io x 100 |
|---|---|
| 30.0 ± 2.2 | W-M |
| 22.1 ± 1.3 | W |
| 3.91 ± 0.07 | M-VS |

and yet more specifically by the lines listed in Table III below:

TABLE III

| Interplanar d-Spacing (A) | Relative Intensity, I/Io x 100 |
|---|---|
| 30.0 ± 2.2 | W-M |
| 22.1 ± 1.3 | W |
| 6.00 ± 0.10 | W-M |
| 4.06 ± 0.07 | W-S |
| 3.91 ± 0.07 | M-VS |

Most specifically, the calcined crystalline material of the invention has an X-ray diffraction pattern which includes the lines listed in Table IV below:

TABLE IV

| Interplanar d-Spacing (A) | Relative Intensity, I/Io x 100 |
|---|---|
| 30.0 ± 2.2 | W-M |
| 22.1 ± 1.3 | W |
| 12.36 ± 0.2 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the diffractometer. From these, the relative intensities, 100 $I/I_o$, where $I_o$ is the intensity of the strongest line or peak, and d (obs.) the interplanar spacing in Angstrom Units (A), corresponding to the recorded lines, were determined. In Tables I-IV, the relative intensities are given in terms of the symbols W=weak, M=medium, S=strong and VS=very strong. In terms of intensities, these may be generally designated as follows:

W = 0 - 20
M = 20 - 40
S = 40 - 60
VS = 60 - 100

It should be understood that this X-ray diffraction pattern is characteristic of all the species of the present crystalline composition. The sodium form as well as other cationic forms reveal substantially the same pattern with some minor shifts in interplanar spacing and variation in relative intensity. Other minor variations can occur, depending on the Y to X, e.g. silicon to aluminum, ratio of the particular sample, as well as its degree of thermal treatment.

The crystalline material of the invention can be prepared from a reaction mixture containing sources of alkali or alkaline earth metal (M), e.g. sodium or potassium, cation, an oxide of trivalent element X, e.g. aluminum, an oxide of tetravalent element Y, e.g. silicon, an organic (R) directing agent, hereinafter more particularly described, and water, said reaction mixture having a composition, in terms of mole ratios of oxides, within the following ranges:

| Reactants | Useful | Preferred |
|---|---|---|
| $YO_2/X_2O_3$ | 10-80 | 10-60 |
| $H_2O/YO_2$ | 5-100 | 10-50 |
| $OH^-/YO_2$ | 0.01-1.0 | 0.1-0.5 |
| $M/YO_2$ | 0.01-2.0 | 0.1-1.0 |
| $R/YO_2$ | 0.05-1.0 | 0.1-0.5. |

It is found that crystallization of the material of the invention is favored if the source of $YO_2$ contains at least 30 wt.% solid $YO_2$, particularly where $YO_2$ is silica. Suitable commercially available sources of silica, include Ultrasil (a precipitated, spray dried silica containing about 90 wt.% silica) and HiSil (a precipitated hydrated $SiO_2$ containing about 87 wt.% silica, about 6 wt.% free $H_2O$ and about 4.5 wt.% bound $H_2O$ of hydration and having a particle size of about 0.02 micron). If another source of silica is used, e.g. Q-Brand (a sodium silicate comprised of about 28.8 wt.% $SiO_2$, 8.9 wt.% $Na_2O$ and 62.3 wt.% $H_2O$), it is found crystallization yields little or none of the crystalline material of this invention and instead impurity phases of other crystal structures, e.g. ZSM-12, are formed. Preferably, therefore, the $YO_2$ source contains at least 30 wt.% solid $YO_2$, preferably silica, and more preferably at least 40 wt.% solid $YO_2$.

The organic directing agent (R) used in synthesizing the present crystalline material from the above reaction mixture is hexamethyleneimine which has the following structural formula:

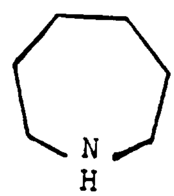

Crystallization of the present crystalline material can be carried out at either static or stirred condition in a suitable reactor vessel, such as for example, polypropylene jars or teflon lined or stainless steel autoclaves. Crystallization is generally conducted at a temperature of 80 °C to 225 °C for 24 hours to 60 days. Thereafter, the crystals are separated from the liquid and recovered.

Crystallization is facilitated by the presence of at least 0.01 percent, preferably 0.10 percent and still more preferably 1 percent, seed crystals (based on total weight) of the desired crystalline product.

Prior to use as a catalyst or an adsorbent, the as synthesized crystalline material should be calcined to remove part or all of any organic constituent.

The crystalline material of the invention can be used as a catalyst in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be peformed. Such component can be in the composition by way of cocrystallization, base exchanged into the composition, impregnated therein or intimately physically admixed therewith. Such component can be impregnated in or on to it such as, for example, by, in the case of platinum, treating the silicate with a solution containing a platinum metal-containing ion. Thus, suitable platinum compounds for this purpose include chloroplatinic acid, platinous chloride and various compounds containing the platinum amine complex.

The crystalline material of the instant invention can be shaped into a wide variety of particle sizes. Generally speaking, the particles can be in the form of a powder, a granule, or a molded product, such as an extrudate. In the latter case, the crystalline material can be extruded before drying or partially dried and then extruded.

The crystalline material of this invention, when employed either as an adsorbent or as a catalyst in an organic compound conversion process should be dehydrated, at least partially. This can be done by heating to a temperature in the range of 200 °C to 595 °C in an atmosphere such as air, nitrogen, etc. and at atmospheric, subatmospheric or superatmospheric pressures for between 30 minutes and 48 hours. Dehydration can also be performed at room temperature merely by placing the silicate in a vacuum, but a longer time is required to obtain a sufficient amount of dehydration.

The crystalline material of this invention can be used to catalyze a wide variety of organic conversion processes including, by way of example, hydration of $C_2$ - $C_7$ olefins, such as propylene, to alcohols and

ethers with reaction conditions including a temperature of 50 to 300°C, preferably 90 to 250°C, a pressure of at least 5 kg/cm$^2$, preferably at least 20 kg/cm$^2$, and a water/olefin mole ratio of 0.1 -30, preferably 0.2 - 15.

As in the case of many catalysts, it may be to incorporate the crystalline material of the invention with another material resistant to the temperatures and other conditions employed in organic conversion processes. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with the new crystal, i.e. combined therewith or present during synthesis of the new crystal, which is active, tends to change the conversion and/or selectivity of the catalyst in certain organic conversion processes. Inactive materials suitably serve as diluents to control the amount of conversion in a given process so that products can be obtained economically and orderly without employing other means for controlling the rate of reaction. These materials may be incorporated into naturally occurring clays, e.g. bentonite and kaolin, to improve the crush strength of the catalyst under commercial operating conditions. Said materials, i.e. clays, oxides, etc., function as hinders for the catalyst. It is desirable to provide a catalyst having good crush strength because in commercial use it is desirable to prevent the catalyst from breaking down into powder-like materials. These clay binders have been employed normally only for the purpose of improving the crush strength of the catalyst.

Naturally occurring clays which can be composited with the new crystal include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the present crystal also include inorganic oxides, notably alumina.

In addition to the foregoing materials, the new crystal can be composited with a porous matrix material such as silica-alumina, silica-magnesia, silica-zirconia, silica-thoria, silica- beryllia, silica-titania as well as ternary compositions such as silica-alumina-thoria, silica-alumina-zirconia silica-alumina-magnesia and silica-magnesia-zirconia.

The relative proportions of finely divided crystalline material and inorganic oxide matrix vary widely, with the crystal content ranging from 1 to 90 percent by weight and more usually, particularly when the composite is prepared in the form of beads, in the range of 2 to 80 weight percent of the composite.

This invention will now be more particularly described with reference to the Examples and the accompanying drawings, in which Figure 1 - 5 are the X-ray diffraction patterns of the calcined products of Examples 1, 3, 4, 5 and 9 respectively. In the examples, whenever sorption data are set forth for comparison of sorptive capacities for water, cyclohexane and/or n-hexane, they were Equilibrium Adsorption values determined as follows:

A weighed sample of the calcined adsorbant was contacted with the desired pure adsorbate vapor in an adsorption chamber, evacuated to less than 1 mm and contacted with 12 Torr of water vapor and 40 Torr of n-hexane or cyclohexane vapor, pressures less than the vapor-liquid equilibrium pressure of the respective adsorbate at 90°C. The pressure was kept constant (within about ± 0.5 mm) by addition of adsorbate vapor controlled by a manostat during the adsorption period, which did not exceed 8 hours. As adsorbate was adsorbed by the new crystal, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant. The synthetic material of this invention always exhibits Equilibrium Adsorption values of greater than 10 wt.% for water vapor, greater than 4.5 wt.%, usually greater than 7 wt.% for cyclohexane vapor and greater than 10 wt.% for n-hexane vapor.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time) compared to that of a highly active silica-alumina cracking catalyst taken as an Alpha of 1 (Rate Constant = 0.016 sec$^{-1}$). The Alpha Test is described in U.S. Patent 3,354,078 and in the Journal of Catalysis, Vol. IV, pp. 522-529 (August 1965). It is noted that intrinsic rate constants for many acid-catalyzed reactions are proportional to the Alpha Value for a particular crystalline silicate catalyst, i.e. the rates for toluene disproportionation, xylene isomerization, alkene conversion and methanol conversion (see "The Active Site of Acidic Aluminosilicate Catalysts," Nature, Vol. 309, No. 5969, pp. 589-591, 14 June 1984).

EXAMPLE 1

Sodium aluminate (43.5% $Al_2O_3$, 32.2% $Na_2O$, 25.6% $H_2O$), 12.86 g , was dissolved in a solution containing 12.8g 50% NaOH solution and 1320 g $H_2O$. To this was added 57.6 g hexamethyleneimine. The resulting solution was added to 109.4 g of Ultrasil, a precipitated, spray-dried silica (about 90% $SiO_2$).

The reaction mixture had the following composition, in mole ratios:

| | |
|---|---|
| $SiO_2/Al_2O_3$ | = 30.0 |
| $OH^-/SiO_2$ | = 0.18 |
| $H_2O/SiO_2$ | = 44.9 |
| $Na/SiO_2$ | = 0.18 |
| $R/SiO_2$ | = 0.35 |

where R is hexamethyleneimine.

The mixture was crystallized in a stainless steel reactor, with stirring, at 150°C for 7 days. The crystalline product was filtered, washed with water and dried at 120°C. After a 20 hour calcination at 538°C, the X-ray diffraction pattern contained the major lines listed in Table V. Figure 1 shows the X-ray diffraction pattern of the calcined product. The sorption capacities of the calcined material were measured to be:

| | |
|---|---|
| $H_2O$ (12 Torr) | 15.2 wt.% |
| Cyclohexane (40 Torr) | 14.6 wt.% |
| n-Hexane (40 Torr) | 16.7 wt.% |

The surface area of the calcined crystalline material was measured to be 494 $m^2/g$.

The chemical composition of the calcined material was determined to be as follows:

| Component | wt% |
|---|---|
| $SiO_2$ | 66.9 |
| $Al_2O_3$ | 5.40 |
| Na | 0.03 |
| N | 2.27 |
| Ash | 76.3 |
| $SiO_2/Al_2O_3$, mole ratio = 21.1 | |

TABLE V

| Degrees 2 Theta | Interplanar d-Spacing (A) | $I/I_o$ |
|---|---|---|
| 2.80 | 31.55 | 25 |
| 4.02 | 21.98 | 10 |
| 7.10 | 12.45 | 96 |
| 7.95 | 11.12 | 47 |
| 10.00 | 8.85 | 51 |
| 12.90 | 6.86 | 11 |
| 14.34 | 6.18 | 42 |
| 14.72 | 6.02 | 15 |
| 15.90 | 5.57 | 20 |
| 17.81 | 4.98 | 5 |
| 19.08 | 4.65 | 2 |
| 20.20 | 4.40 | 20 |
| 20.91 | 4.25 | 5 |
| 21.59 | 4.12 | 20 |
| 21.92 | 4.06 | 13 |
| 22.67 | 3.92 | 30 |
| 23.70 | 3.75 | 13 |
| 25.01 | 3.56 | 20 |
| 26.00 | 3.43 | 100 |
| 26.96 | 3.31 | 14 |
| 27.75 | 3.21 | 15 |
| 28.52 | 3.13 | 10 |
| 29.01 | 3.08 | 5 |
| 29.71 | 3.01 | 5 |
| 31.61 | 2.830 | 5 |
| 32.21 | 2.779 | 5 |
| 33.35 | 2.687 | 5 |
| 34.61 | 2.592 | 5 |

EXAMPLE 2

A portion of the calcined crystalline product of Example 1 was tested in the Alpha Test, and found to have an Alpha Value of 224.

EXAMPLES 3 - 5

Three separate synthesis reaction mixtures were prepared with compositions indicated in Table VI. The mixtures were prepared with sodium aluminate, sodium hydroxide, Ultrasil, hexamethyleneimine (R) and water. The mixtures were maintained at 150°C, 143°C and 150°C, respectively, for 7, 8 and 6 days, respectively, in a stainless steel, stirred (350 rpm) autoclave at autogenous pressure. Solids were separated from any unreacted components by filtration and then water washed, followed by drying at 120°C. The product crystals were analyzed by X-ray diffraction, sorption, surface area and chemical analyses. The products were found to be the new crystalline material of the present invention. Results of sorption, surface area and chemical analyses are also presented in Table VI. The X-ray diffraction patterns of the calcined (538°C for 3 hours) products of Examples 3, 4, and 5 are presented in Figures 2, 3, and 4, respectively. The sorption and surface area measurements were of the calcined product.

TABLE VI

| Example Number | 3 | 4 | 5 |
|---|---|---|---|
| Synthesis Mixture, mole ratios | | | |
| $SiO_2/Al_2O_3$ | 30.0 | 30.0 | 30.0 |
| $OH^-/SiO_2$ | 0.18 | 0.18 | 0.18 |
| $H_2O/SiO_2$ | 19.4 | 19.4 | 44.9 |
| $Na/SiO_2$ | 0.18 | 0.18 | 0.18 |
| $R/SiO_2$ | 0.35 | 0.35 | 0.35 |
| Product Composition, Wt.% | | | |
| $SiO_2$ | 64.3 | 68.5 | 74.5 |
| $Al_2O_3$ | 4.85 | 5.58 | 4.87 |
| Na | 0.08 | 0.05 | 0.01 |
| N | 2.40 | 2.33 | 2.12 |
| Ash | 77.1 | 77.3 | 78.2 |
| $SiO_2/Al_2O_3$, mole ratio | 22.5 | 20.9 | 26.0 |
| Adsorption, wt.% | | | |
| $H_2O$ | 14.9 | 13.6 | 14.6 |
| Cyclohexane | 12.5 | 12.2 | 13.6 |
| n-Hexane | 14.6 | 16.2 | 19.0 |
| Surface Area, $m^2/g$ | 481 | 492 | 487 |

EXAMPLE 6

Quantities of the calcined (538°C for 3 hours) crystalline silicate products of Examples 3, 4 and 5 were tested in the Alpha Test, and found to have Alpha Values of 227, 180 and 187, respectively.

EXAMPLE 7

A calcined sample of the crystalline silicate of Example 4 was impregnated with $Pt(NH_3)_4Cl_2$ solution to about 1 wt.% Pt. This material was then heated in air at 349°C for 3 hours.

EXAMPLE 8

One gram of the resulting product from Example 7 was charged as catalyst to a small reactor with a preheater and imbedded thermocouple. The catalyst was then heated at 482°C with flowing hydrogen to reduce the Pt component. Normal decane and hydrogen were charged over the catalyst to give 0.4 $hr^{-1}$ WHSV in decane and a hydrogen/hydrocarbon molar ratio of 100/1. The reaction was carried out in the temperature range of 130-250°C and atmospheric pressure.

The results of this experiment are summarized in Table VII, together with results of the same experiment, but with the crystalline material being changed to ZSM-5 (U.S. Patent 3,702,886), ZSM-11 (U.S. Patent 3,709,979) and Zeolite Beta (U.S. Patent 3,308,069), presented for comparative purposes. It is observed that the crystalline silicate of the present invention is a very active catalyst for n-decane hydroconversion and has good isomerization activity. In Table VII, "5MN/2MN" is the molecular ratio of 5-methylnonane/2-methylnonane. Due to the position of its methyl group, 5-methylnonane offers slightly higher steric hindrance to enter zeolitic pores. The 5MN/2MN ratio provides information about the porosity of the zeolite being tested.

TABLE VII

| Catalyst Zeolite | $SiO_2/Al_2O_3$ Mole Ratio of Zeolite | Temp. (°C) for 50% Conversion | % Isom. at 50%. Conversion | 5MN/2MN 5% Isom. | 5MN/2MN 20% Isom. |
|---|---|---|---|---|---|
| Example 7 | 20.9 | 174 | 70 | 0.15 | 0.24 |
| ZSM-5 | 50 | 187 | 65 | 0.11 | 0.15 |
| ZSM-11 | 40 | 187 | 94 | 0.36 | 0.41 |
| ZSM-23 | 85 | 211 | 90 | 0.27 | 0.28 |
| Beta | 30 | 189 | 76 | 0.68 | 0.59 |

EXAMPLE 9

To demonstrate a larger preparation of the crystalline material of this invention, 1200 g of hexamethyleneimine was added to a solution containing 268 g of sodium aluminate, 267 g of 50% NaOH solution and 11,800 g of $H_2O$. To the combined solution was added 2,280 g of Ultrasil silica. The mixture was crystallized with agitation (about 200 rpm) at 145 °C in a 5 gallon reactor. Crystallization time was 59 hours. The product was water washed and dried at 120 °C.

The X-ray diffraction pattern of the calcined (538 °C) product crystals is presented in Figure 5 and demonstrates the product to be the crystalline material of this invention. Product chemical composition, surface area and adsorption analyses results were as follows:

TABLE VIII

| | |
|---|---|
| Product Composition | |
| C | 12.1 wt.% |
| N | 1.98 wt.% |
| Na | 640 ppm |
| $Al_2O_3$ | 5.0 wt.% |
| $SiO_2$ | 74.9 wt.% |
| $SiO_2/Al_2O_3$, mole ratio | 25.4 |
| Adsorption, wt.% | |
| Cyclohexane | 9.1 |
| n-Hexane | 14.9 |
| $H_2O$ | 16.8 |
| Surface Area, $m^2/g$ | 479 |

EXAMPLE 10

A 25 g quantity of solid crystal product from Example 9 was calcined in a flowing nitrogen atmosphere at 538 °C for 5 hours, followed by purging with 5% oxygen gas (balance $N_2$) for another 16 hours at 538 °C.

Individual 3 g samples of the calcined material were ion-exchanged with 100 ml of 0.1N TEABr, TPABr and $LaCl_3$ solution separately. Each exchange was carried out at ambient temperature for 24 hours and repeated three times. The exchanged samples were collected by filtration, water-washed to be halide free and dried. The compositions of the exchanged samples are tabulated below demonstrating the exchange capacity of the present crystalline silicate for different ions.

| Exchange Ions | TEA | TPA | La |
|---|---|---|---|
| Ionic Composition, wt.% | | | |
| Na | 0.095 | 0.089 | 0.063 |
| N | 0.30 | 0.38 | 0.03 |
| C | 2.89 | 3.63 | - |
| La | - | - | 1.04 |

EXAMPLE 11

The above La-exchanged sample was sized to 14 to 25 mesh and then calcined in air at 538°C for 3 hours. The calcined material had an Alpha Value of 173.

EXAMPLE 12

The calcined sample of La-exchanged material from Example 11 was severely steamed at 649°C in 100% steam for 2 hours. The steamed sample had an Alpha Value of 22, demonstrating that the crystalline silicate hereof has very good stability under severe hydrothermal treatment.

EXAMPLE 13

To prepare the present crystal with X comprising boron, 17.5 g quantity of boric acid was added to a solution containing 6.75 g of 45% KOH solution and 290 g $H_2O$. To this was added 57.8 g of Ultrasil silica, and the mixture was thoroughly homogenized. A 26.2 g quantity of hexamethyleneimine was added to the mixture.

The reaction mixture had the following composition in mole ratios:

| | |
|---|---|
| $SiO_2/B_2O_3$ | = 6.1 |
| $OH^-/SiO_2$ | = 0.06 |
| $H_2O/SiO_2$ | = 19.0 |
| $K/SiO_2$ | = 0.06 |
| $R/SiO_2$ | = 0.30 |

where R is hexamethyleneimine.

The mixture was crystallized in a stainless steel reactor, with agitation, at 150°C for 8 days. The crystalline product was filtered, washed with water and dried at 120°C. A portion of the product was calcined for 6 hours at 540°C and found to have the following sorption capacities:

| | |
|---|---|
| $H_2O$ (12 Torr) | 11.7 wt.% |
| Cyclohexane (40 Torr) | 7.5 wt.% |
| n-Hexane (40 Torr) | 11.4 wt.% |

The surface area of the calcined crystalline material was measured to be 405 $m^2/g$.

The chemical composition of the calcined material was determined to be as follows:

| | |
|---|---|
| N | 1.94 wt.% |
| Na | 175 ppm |
| K | 0.60 wt.% |
| Boron | 1.04 wt.% |
| $Al_2O_3$ | 920 ppm |
| $SiO_2$ | 75.9 wt.% |
| Ash | 74.11 wt.% |
| $SiO_2/Al_2O_3$, molar ratio | = 1406 |
| $SiO_2/(Al+B)_2O_3$, molar ratio | = 25.8 |

EXAMPLE 14

A portion of the calcined crystalline product of Example 13 was treated with $NH_4Cl$ and again calcined. The final crystalline product was tested in the Alpha Test and found to have an Alpha Value of 1.

EXAMPLE 15

To prepare the present crystalline material with X comprising boron, 35.0 g quantity of boric acid was added to a solution of 15.7 g of 50% NaOH solution and 1160 g $H_2O$. To this solution was added 240 g of HiSil silica followed by 105 g of hexamethyleneimine. The reaction mixture had the following composition in mole ratios:

| | |
|---|---|
| $SiO_2/B_2O_3$ | = 12.3 |
| $OH^-/SiO_2$ | = 0.056 |
| $H_2O/SiO_2$ | = 18.6 |
| $Na/SiO_2$ | = 0.056 |
| $R/SiO_2$ | = 0.30 |

where R is hexamethyleneimine.

The mixture was crystallized in a stainless steel reactor, with agitation, at 300°C for 9 days. The crystalline product was filtered, washed with water and dried at 120°C. The sorption capacities of the calcined material (6 hours at 540°C) were measured to be:

| | |
|---|---|
| $H_2O$ (12 Torr) | 14.4 wt.% |
| Cyclohexane (40 Torr) | 4.6 wt.% |
| n-Hexane (40 Torr) | 14.0 wt.% |

The surface area of the calcined crystalline material was measured to be 438 $m^2/g$.

The chemical composition of the calcined material was determined to be as follows:

| Component | Wt.% |
|---|---|
| N | 2.48 |
| Na | 0.06 |
| Boron | 0.83 |
| $Al_2O_3$ | 0.50 |
| $SiO_2$ | 73.4 |
| $SiO_2/Al_2O_3$, molar ratio | = 249 |
| $SiO_2/(Al+B)_2O_3$, molar ratio | = 28.2 |

EXAMPLE 16

A portion of the calcined crystalline product of Example 15 was tested in the Alpha Test and found to have an Alpha Value of 5.

EXAMPLE 17

To demonstrate the importance of employing a silica source containing at least 30% solid silica in the method of this invention, Example 3 was repeated, but with Q-Brand sodium silicate (containing only about 29 wt.% solid silica) being used as the source of silica. In this example, 67.6 g of aluminum sulfate was dissolved in a solution of 38.1 g $H_2SO_4$ (96.1%) and 400 g water. The resulting solution was mixed with 120 g of hexamethyleneimine and added to a mixture of 712.5 g Q-Brand sodium silicate (28.8% $SiO_2$ and 8.9% $Na_2O$) and 351 g water. The resulting mixture, having the following composition expressed in mole ratios:

| | |
|---|---|
| $SiO_2/Al_2O_3$ | = 30.0 |
| $OH^-/SiO_2$ | = 0.18 |
| $H_2O/SiO_2$ | = 19.4 |
| $Na/SiO_2$ | = 0.60 |
| $R/SiO_2$ | = 0.35 |

was thoroughly mixed and crystallized with stirring in a stainless steel reactor at 246 °C for 8 days. The product solids were separated from unreacted components by filtration and then water washed, followed by drying at 120 °C. The product was analyzed by X-ray diffraction and found to be a mixture of amorphous material, magadiite and mordenite. No crystals of the present invention were found.

EXAMPLE 18

In this Example, the propylene hydration properties of the crystalline material of the invention were compared with those of ZSM-12 and PSH-3 produced according to Example 4 of U.S. Patent No. 4439409.

The crystalline material of the invention was prepared by adding 15.9 parts by weight of hexamethyleneimine to a mixture containing 3.5 parts 50% NaOH, 3.5 parts sodium aluminate, 30.1 parts Ultrasil VN3 silica and 156 parts deionized $H_2O$. The reaction mixture was heated directly to 290 °F (143 °C) and stirred in an autoclave at that temperature to effect crystallization. After full crystallinity was achieved, the resulting crystals were separated from remaining liquid by filtration, washed with water and dried. A portion of the crystals was combined with alumina to form a mixture of 65 parts, by weight, zeolite and 35 parts alumina. Enough water was added to this mixture so that the resulting catalyst could be formed into extrudates. This catalyst was activated by calcining in nitrogen at 1000 °F (540 °C) followed by aqueous 1.0N ammonium nitrate exchange and calcining in air at 1000 - 1200 °F (540 - 650 °C).

The ZSM-12 was prepared by adding 1 part by weight ZSM-12 seeds to a mixture containing 41.5 parts Hi-Sil 233 silica, 67.7 parts 50% tetraethyl ammonium bromide, 7.0 parts 50% NaOH and 165.4 parts deionized $H_2O$. The reaction mixture was heated directly to 280 °F (138 °C) and stirred in an autoclave at that temperature for crystallization. After full crystallinity was achieved, the resulting crystals were separated from remaining liquid by filtration, washed with water and dried. A portion of the crystals was combined with alumina to form a mixture of 65 parts, by weight, zeolite and 35 parts alumina. Enough water was added to this mixture so that the resulting catalyst could be formed inot extrudates. This catalyst was activated by calcining in nitrogen at 1000 °F (540 °C), followed by aqueous 1.0N ammonium nitrate exchange and calcining at 1200 °F (650 °C).

Propylene hydration was conducted at a temperature of 330 °F (166 °C), a pressure of 1000 psig (7000 kPa) and WHSV of propylene of 0.6. The results are 2 days in steam are compared in Table IX below.

TABLE IX

| | PSH-3 | Material of the Invention | ZSM-12 |
|---|---|---|---|
| % Propylene Conversion | 1.9 | 27.8 | 10.8 |
| % Water Conversion | 5.7 | 33.7 | 8.1 |
| IPA Selectivity | 88.7 | 76.7 | 74.6 |
| DIPE Selectivity | 2.9 | 22.3 | 23.1 |
| Oligomer Selectivity | 8.4 | 1.2 | 2.3 |
| IPA = isopropyl alcohol DIPE = diisopropyl ether | | | |

The above results clearly show the advantageous properties of the material of the invention as compared to both PSH-3 and ZSM-12 in achieving a higher rate of conversion coupled with good selectivity to diisopropyl benzene and low production of propylene oligomers.

## Claims

**1.** A synthetic porous crystalline material characterized by an x-ray diffraction pattern including values substantially as set forth in Table I below and having equilibrium adsorption capacities of greater than 10 wt.% for water vapor, greater than 4.5 wt.% for cyclohexane vapor, and greater than 10 wt.% for n-hexane vapor:

TABLE I

| Interplanar d-Spacing (A) | Relative Intensity, I/Iox100 |
|---|---|
| 30.0 ± 2.2 | W-M |
| 22.1 ± 1.3 | W |

**2.** The synthetic porous crystalline material of claim 1 characterized by an x-ray diffraction pattern including values substantially as set forth in Table II below:

TABLE II

| Interplanar d-Spacing (A) | Relative Intensity, I/Iox100 |
|---|---|
| 30.0 ± 2.2 | W-M |
| 22.1 ± 1.3 | W |
| 3.91 ± 0.07 | M-VS |

**3.** The synthetic porous crystalline material of claim 1 characterized by an x-ray diffraction pattern including values substantially as set forth in Table III below:

TABLE III

| Interplanar d-Spacing (A) | Relative Intensity, I/Iox100 |
|---|---|
| 30.0 ± 2.2 | W-M |
| 22.1 ± 1.3 | W |
| 6.00 ± 0.10 | W-M |
| 4.06 ± 0.07 | W-S |
| 3.91 ± 0.07 | M-VS |

**4.** The synthetic porous crystalline material of claim 1 characterized by an x-ray diffraction pattern including values substantially as set forth in Table IV below:

TABLE IV

| Interplanar d-Spacing (A) | Relative Intensity, I/Iox100 |
|---|---|
| 30.0 ± 2.2 | W-M |
| 22.1 ± 1.3 | W |
| 12.36 ± 0.2 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

**5.** The crystalline material of any preceding claim having a composition comprising the molar relationship

$X_2O_3:(n)YO_2$,

wherein n is at least 10, X is a trivalent element and Y is a tetravalent element.

**6.** The crystalline material of claim 5 wherein X comprises aluminum and Y comprises silicon.

**7.** The crystalline material of claim 6 wherein n is from 20 to 40.

**8.** A method for preparing the synthetic crystalline material of claim 5, said method comprising preparing a reaction mixture capable of forming said material upon crystallization, said reaction mixture containing sufficient amounts of alkali or alkaline earth metal cations, a source of tetravalent Y oxide containing at least 30 wt.% solid $YO_2$, a source of trivalent X oxide, water and hexamethyleneimine, and maintaining said reaction mixture under sufficient crystallization conditions until crystals of said material are formed.

**9.** The method of claim 8 wherein said reaction mixture has a composition in terms of mole ratios within the following ranges:

| | |
|---|---|
| $YO_2/X_2O_3$ | = 10 to 80 |
| $H_2O/YO_2$ | = 5 to 100 |
| $OH^-/YO_2$ | = 0.01 to 1.0 |
| $M/YO_2$ | = 0.01 to 2.0 |
| $R/YO_2$ | = 0.05 to 1.0 |

wherein R represents hexamethyleneimine and M represents alkali or alkaline earth metal.

**10.** The method of claim 8 wherein said reaction mixture has a composition in terms of mole ratios within the following ranges:

| | |
|---|---|
| $YO_2/X_2O_3$ | = 10 to 60 |
| $H_2O/YO_2$ | = 10 to 50 |
| $OH^-/YO_2$ | = 0.1 to 0.5 |
| $M/YO_2$ | = 0.1 to 1.0 |
| $R/YO_2$ | = 0.1 to 0.5. |

**Patentansprüche**

**1.** Synthetisches poröses kristalline Material, gekennzeichnet durch ein Röntgenbeugungsdiagramm, das im wesentlichen die in der nachfolgenden Tabelle I aufgeführten Werte umfaßt und Gleichgewichts-Adsorptionkapazitäten von mehr als 10 Gew.-% für Wasserdampf, mehr als 4,5 Gew.-% für Cyclohexandampf und mehr als 10 Gew.-% für n-Hexandampf aufweist:

Tabelle I

| d-Netzebenenabstand (Å) | Relative Intensität, I/Iox100 |
|---|---|
| 30,0 ± 2,2 | W-M |
| 22,1 ± 1,3 | W |

**2.** Synthetisches poröses kristallines Material nach Anspruch 1, gekennzeichnet durch ein Röntgenbeugungsdiagramm, das im wesentlichen die in der nachfolgenden Tabelle II aufgeführten Werte umfaßt:

Tabelle II

| d-Netzebenenabstand (Å) | Relative Intensität, I/Iox100 |
|---|---|
| 30,0 ± 2,2 | W-M |
| 22,1 ± 1,3 | W |
| 3,91 ± 0,07 | M-VS |

**3.** Synthetisches poröses kristallines Material nach Anspruch 1, gekennzeichnet durch ein Röntgenbeugungsdiagramm, das im wesentlichen die in der nachfolgenden Tabelle III aufgeführten Werte umfaßt:

Tabelle III

| d-Netzebenenabstand (Å) | Relative Intensität, I/Iox100 |
|---|---|
| 30,0 ± 2,2 | W-M |
| 22,1 ± 1,3 | W |
| 6,00 ± 0,10 | W-M |
| 4,06 ± 0,07 | W-S |
| 3,91 ± 0,07 | M-VS |

**4.** Synthetisches poröses kristallines Material nach Anspruch 1, gekennzeichnet durch ein Röntgenbeugungsdiagramm, das im wesentlichen die in der nachfolgenden Tabelle IV aufgeführten Werte umfaßt:

Tabelle IV

| d-Netzebenenabstand (Å) | Relative Intensität, I/Iox100 |
|---|---|
| 30,0 ± 2,2 | W-M |
| 22,1 ± 1,3 | W |
| 12,36 ± 0,2 | M-VS |
| 11,03 ± 0,2 | M-S |
| 8,83 ± 0,14 | M-VS |
| 6,86 ± 0,14 | W-M |
| 6,18 ± 0,12 | M-VS |
| 6,00 ± 0,10 | W-M |
| 5,54 ± 0,10 | W-M |
| 4,92 ± 0,09 | W |
| 4,64 ± 0,08 | W |
| 4,41 ± 0,08 | W-M |
| 4,25 ± 0,08 | W |
| 4,10 ± 0,07 | W-S |
| 4,06 ± 0,07 | W-S |
| 3,91 ± 0,07 | M-VS |
| 3,75 ± 0,06 | W-M |
| 3,56 ± 0,06 | W-M |
| 3,42 ± 0,06 | VS |
| 3,30 ± 0,05 | W-M |
| 3,20 ± 0,05 | W-M |
| 3,14 ± 0,05 | W-M |
| 3,07 ± 0,05 | W |
| 2,99 ± 0,05 | W |
| 2,82 ± 0,05 | W |
| 2,78 ± 0,05 | W |
| 2,68 ± 0,05 | W |
| 2,59 ± 0,05 | W |

**5.** Kristallines Material nach einem der vorstehenden Ansprüche mit einer Zusammensetzung, die das molare Verhältnis umfaßt:

$X_2O_3$: (n) $YO_2$,

worin n mindestens 10 beträgt, X ein dreiwertiges Element und Y ein vierwertiges Element darstellen.

**6.** Kristallines Material nach Anspruch 5, worin X Aluminium und Y Silicium umfassen.

7. Kristallines Material nach Anspruch 6, worin n 20 bis 40 beträgt.

8. Verfahren zur Herstellung des synthetischen kristallinen Materials nach Anspruch 5, wobei dieses Verfahren die Herstellung einer Reaktionsmischung, die bei der Kristallisation dieses Marial bilden kann, wobei diese Reaktionsmischung ausreichende Mengen von Alkali- oder Erdalkalimetallkationen, eine Quelle eines vierwertigen Y-Oxids, das mindestens 30 Gew.-% festes $YO_2$ enthält, eine Quelle eines dreiwertigen X-Oxids, Wasser und Hexamethylenimin enthält, und das Halten dieser Reaktionsmischung bei ausreichenden Kristallisationsbedingungen umfaßt, bis Kristalle dieses Materials gebildet sind.

9. Verfahren nach Anspruch 8, worin die Reaktionsmischung eine auf die Molverhältnisse bezogene Zusammensetzung innerhalb der folgenden Bereiche aufweist:

| | |
|---|---|
| $YO_2/X_2O_3$ | = 10 bis 80 |
| $H_2O/YO_2$ | = 5 bis 100 |
| $OH^-/YO_2$ | = 0,01 bis 1,0 |
| $M/YO_2$ | = 0,01 bis 2,0 |
| $R/YO_2$ | = 0,05 bis 1,0 |

worin R Hexamethylenimin und M ein Alkali- oder Erdalkalimetall darstellen.

10. Verfahren nach Anspruch 8, worin die Reaktionsmischung eine auf die Molverhältnisse bezogene Zusammensetzung innerhalb der folgenden Bereiche aufweist:

| | |
|---|---|
| $YO_2/X_2O_3$ | = 10 bis 60 |
| $H_2O/YO_2$ | = 10 bis 50 |
| $OH^-/YO_2$ | = 0,1 bis 0,5 |
| $M/YO_2$ | = 0,1 bis 1,0 |
| $R/YO_2$ | = 0,1 bis 0,5 |

**Revendications**

1. Matériau cristallin poreux de synthèse, caractérisé par un spectre de diffraction des rayons X présentant sensiblement les valeurs indiquées dans le tableau I ci-après et ayant des capacités d'adsorption à l'équilibre qui sont supérieures à 10% en poids pour la vapeur d'eau, supérieures à 4,5% en poids pour la vapeur de cyclohexane et supérieures à 10% en poids pour la vapeur de n-hexane:

Tableau I

| Distance d'espacement d entre les plans (Å) | Intensité relative, I/Io x 100 |
|---|---|
| 30,0 ± 2 2 | W-M |
| 22,1 ± 1 3 | W |

2. Matériau cristallin poreux de synthèse selon la revendication 1, caractérisé par un spectre de diffraction des rayons X présentant sensiblement les valeurs indiquées dans le tableau II ci-après:

Tableau II

| Distance d'espacement d entre les plans (Å) | Intensité relative, I/Io x 100 |
|---|---|
| 30,0 ± 2,2 | W-M |
| 22,1 ± 1 3 | W |
| 3,91 ± 0,07 | M-VS |

**3.** Matériau cristallin poreux de synthèse selon la revendication 1, caractérisé par un spectre de diffraction des rayons X présentant sensiblement les valeurs indiquées dans le tableau III ci-après:

Tableau III

| Distance d'espacement d entre les plans (Å) | Intensité relative, I/Io x 100 |
|---|---|
| 30,0 ± 2 2 | W-M |
| 22,1 ± 1,3 | W |
| 6,00 ± 0,10 | W-M |
| 4,06 ± 0,07 | W-S |
| 3,91 ± 0,07 | M-VS |

**4.** Matériau cristallin poreux de synthèse selon la revendication 1, caractérisé par un spectre de diffraction des rayons X comprenant pratiquement les valeurs indiquées dans le tableau IV ci-après:

Tableau IV

| Distance d'espacement d entre les plans (Å) | Intensité relative, I/Io x 100 |
|---|---|
| 30,0 ± 2,2 | W-M |
| 22,1 ± 1,3 | W |
| 12,36 ± 0,2 | M-VS |
| 11,03 ± 0,2 | M-S |
| 8,83 ± 0,14 | M-VS |
| 6,86 ± 0,14 | W-M |
| 6,18 ± 0,12 | M-VS |
| 6,00 ± 0,10 | W-M |
| 5,54 ± 0,10 | W-M |
| 4,92 ± 0,09 | W |
| 4,64 ± 0,08 | W |
| 4,41 ± 0,08 | W-M |
| 4,25 ± 0,08 | W |
| 4,10 ± 0,07 | W-S |
| 4,06 ± 0,07 | W-S |
| 3,91 ± 0,07 | M-VS |
| 3,75 ± 0,06 | W-M |
| 3,56 ± 0,06 | W-M |
| 3,42 ± 0,06 | VS |
| 3,30 ± 0,05 | W-M |
| 3,20 ± 0,05 | W-M |
| 3,14 ± 0,05 | W-M |
| 3,07 ± 0,05 | W |
| 2,99 ± 0,05 | W |
| 2,82 ± 0,05 | W |
| 2,78 ± 0,05 | W |
| 2,68 ± 0,05 | W |
| 2,59 ± 0,05 | W |

**5.** Matériau cristallin selon l'une quelconque des revendications précédentes, ayant une composition présentant la relation molaire:

$X_2O_3$ / (n) $YO_2$,

dans laquelle:
n est au moins égal à 10, X est un élément trivalent et Y est un élément tétravalent.

**6.** Matériau cristallin selon la revendication 5, dans lequel X comprend l'aluminium et Y comprend le silicium.

**7.** Matériau cristallin selon la revendication 6, dans lequel n est un nombre de 20 à 40.

**8.** Procédé pour préparer un matériau cristallin synthétique selon la revendication 5, qui comprend la formation d'un mélange réactionnel capable de former ce matériau par cristallisation, ce mélange réactionnel contenant des quantités suffisantes de cations de métaux alcalins ou alcalinoterreux, une source d'oxyde de Y tétravalent contenant au moins 30% en poids de $YO_2$ solide, une source d'oxyde de X trivalent, de l'eau et de l'hexaméthylène imine, et le maintien de ce mélange réactionnel dans des conditions de cristallisation suffisantes jusqu'à formation de cristaux de ce matériau.

**9.** Procédé selon la revendication 8, dans lequel ce mélange réactionnel a une composition, exprimée en termes de rapports molaires, qui est comprise dans les gammes suivantes:

| | |
|---|---|
| $YO_2/X_2O_3$ | 10 à 80 |
| $H_2O/YO_2$ | 5 à 100 |
| $OH^-/YO_2$ | 0,01 à 1,0 |
| $M/YO_2$ | 0,01 à 2,0 |
| $R/YO_2$ | 0,05 à 1,0 |

dans lequel:

R représente l'hexaméthylène imine et

M est un métal alcalin ou un métal alcalino-terreux.

**10.** Procédé selon la revendication 8, dans lequel ce mélange réactionnel a une composition, exprimée en termes de rapports molaires, comprise dans les gammes suivantes:

| | |
|---|---|
| $YO_2/X_2O_3$ | 10 à 60 |
| $H_2O/YO_2$ | 10 à 50 |
| $OH^-/YO_2$ | 0,1 à 0,5 |
| $M/YO_2$ | 0,1 à 1,0 |
| $R/YO_2$ | 0,1 à 0,5 |

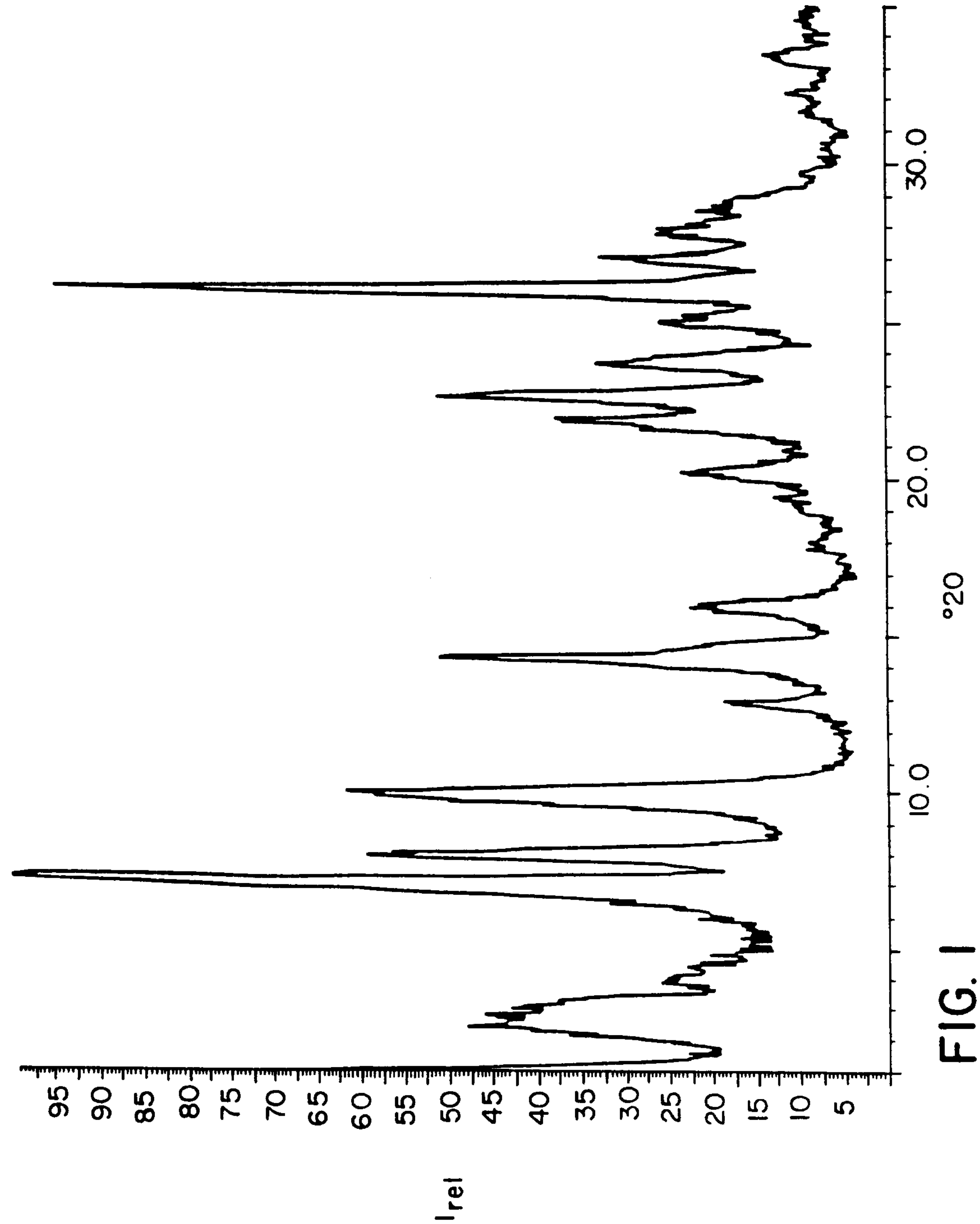

I rel
95
90
85
80
75
70
65
60
55
50
45
40
35
30
25
20
15
10
5
10.0
20.0
30.0
°2θ


FIG. I

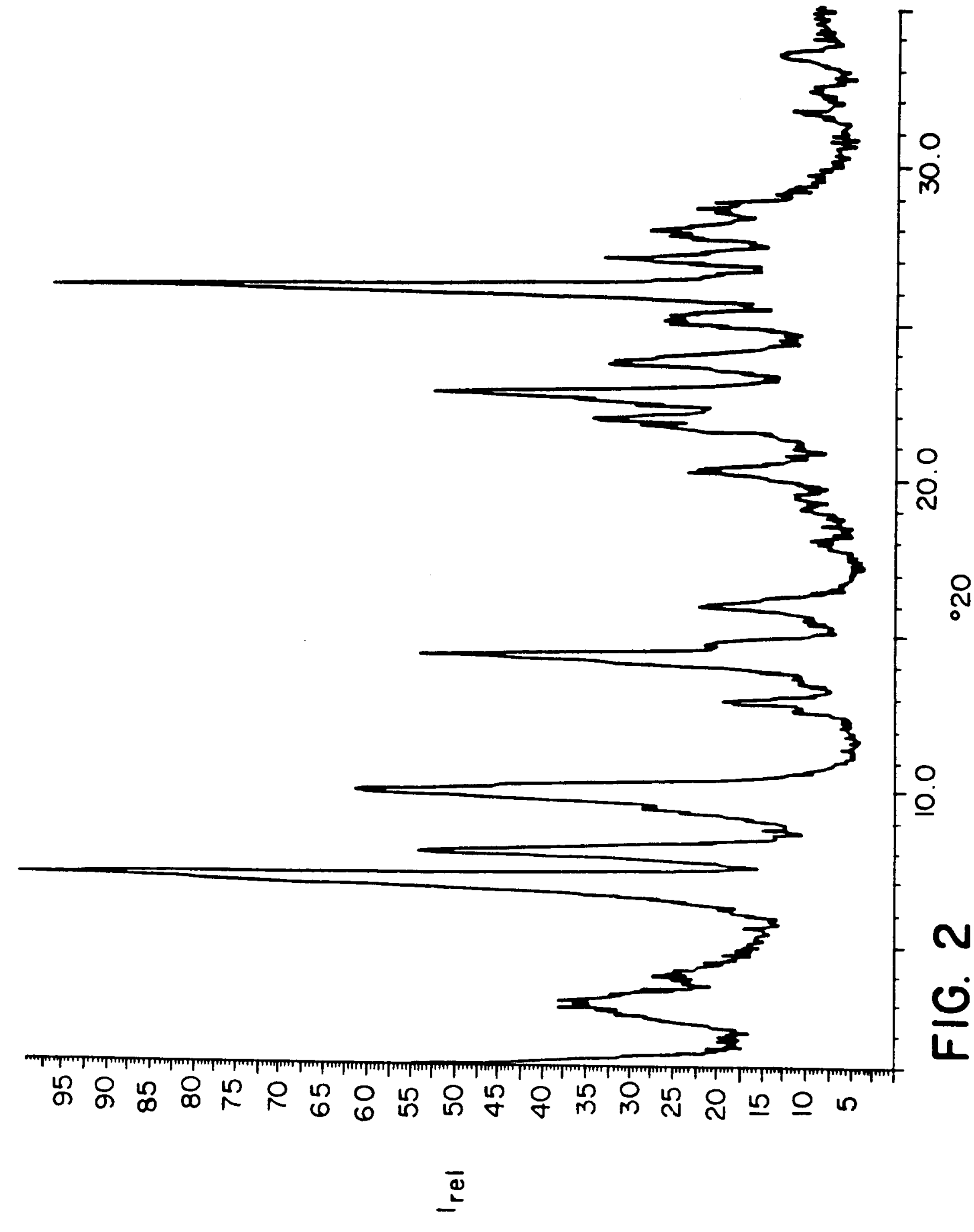
95
90
85
80
75
70
65
60
55
50
45
40
35
30
25
20
15
10
5
Irel
10.0
20.0
30.0
°2θ

FIG. 2

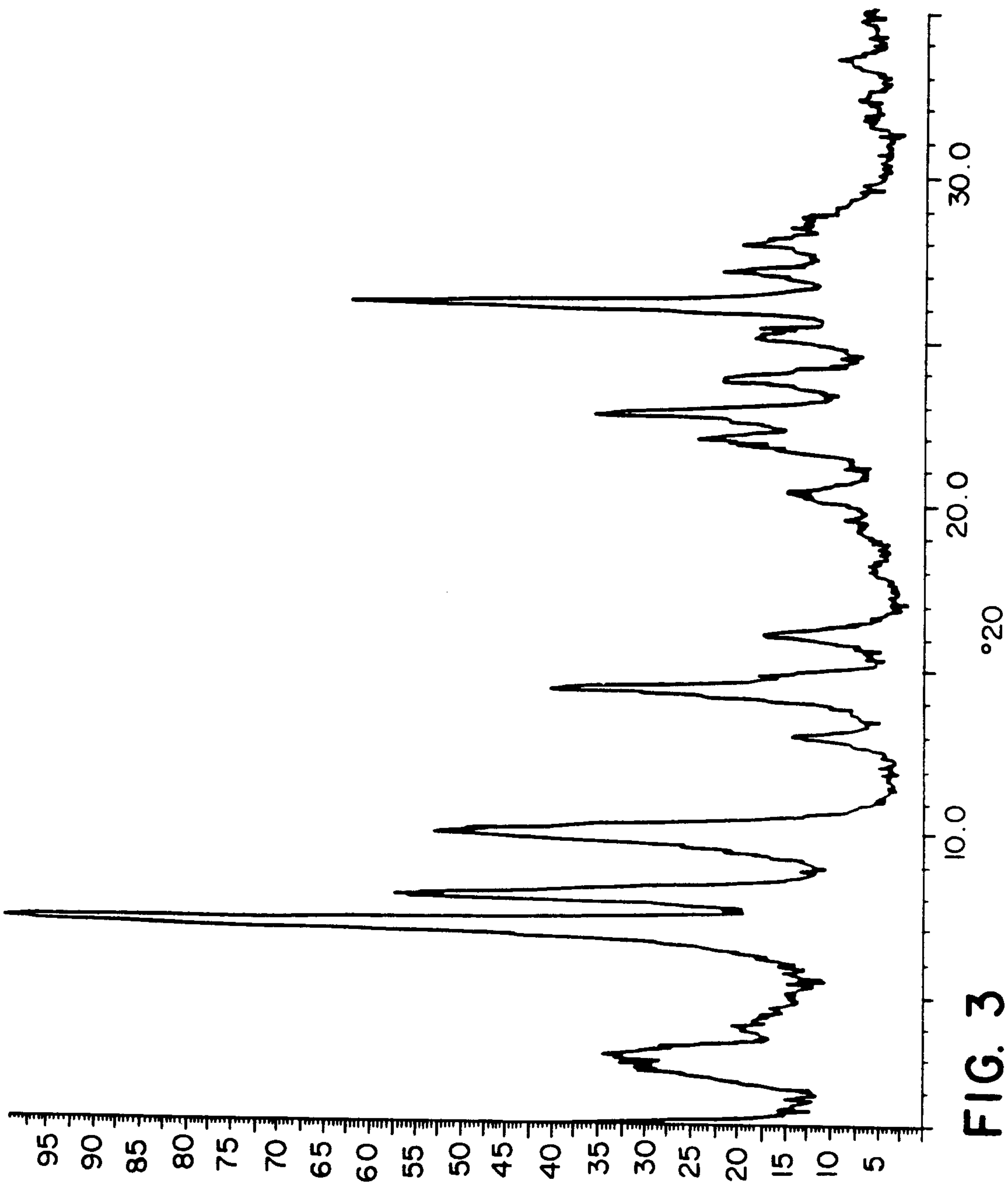
I rel
95
90
85
80
75
70
65
60
55
50
45
40
35
30
25
20
15
10
5
10.0
20.0
30.0
°2θ

FIG. 3

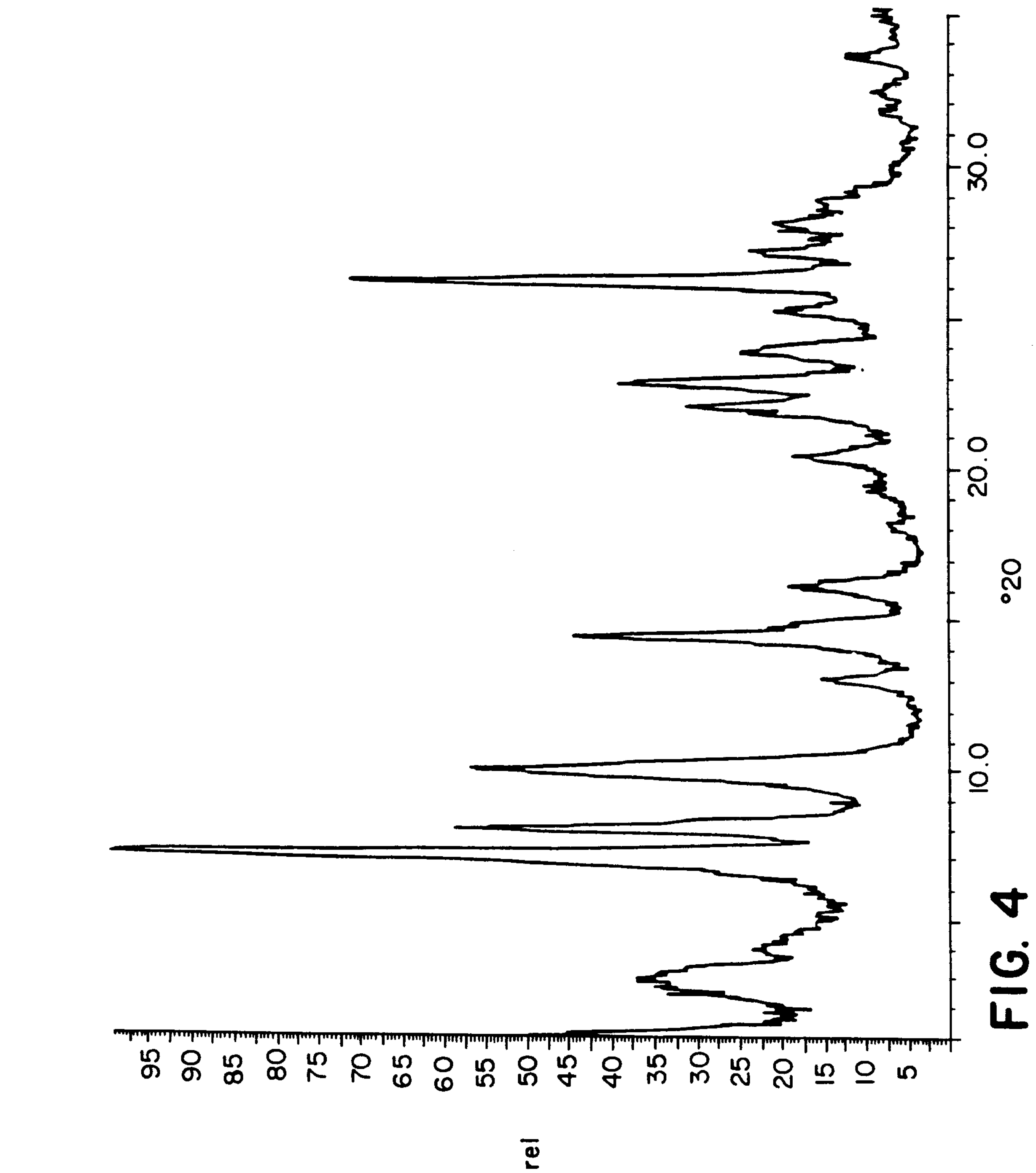
$I_{rel}$
95
90
85
80
75
70
65
60
55
50
45
40
35
30
25
20
15
10
5
10.0
20.0
30.0
°2θ

FIG. 4

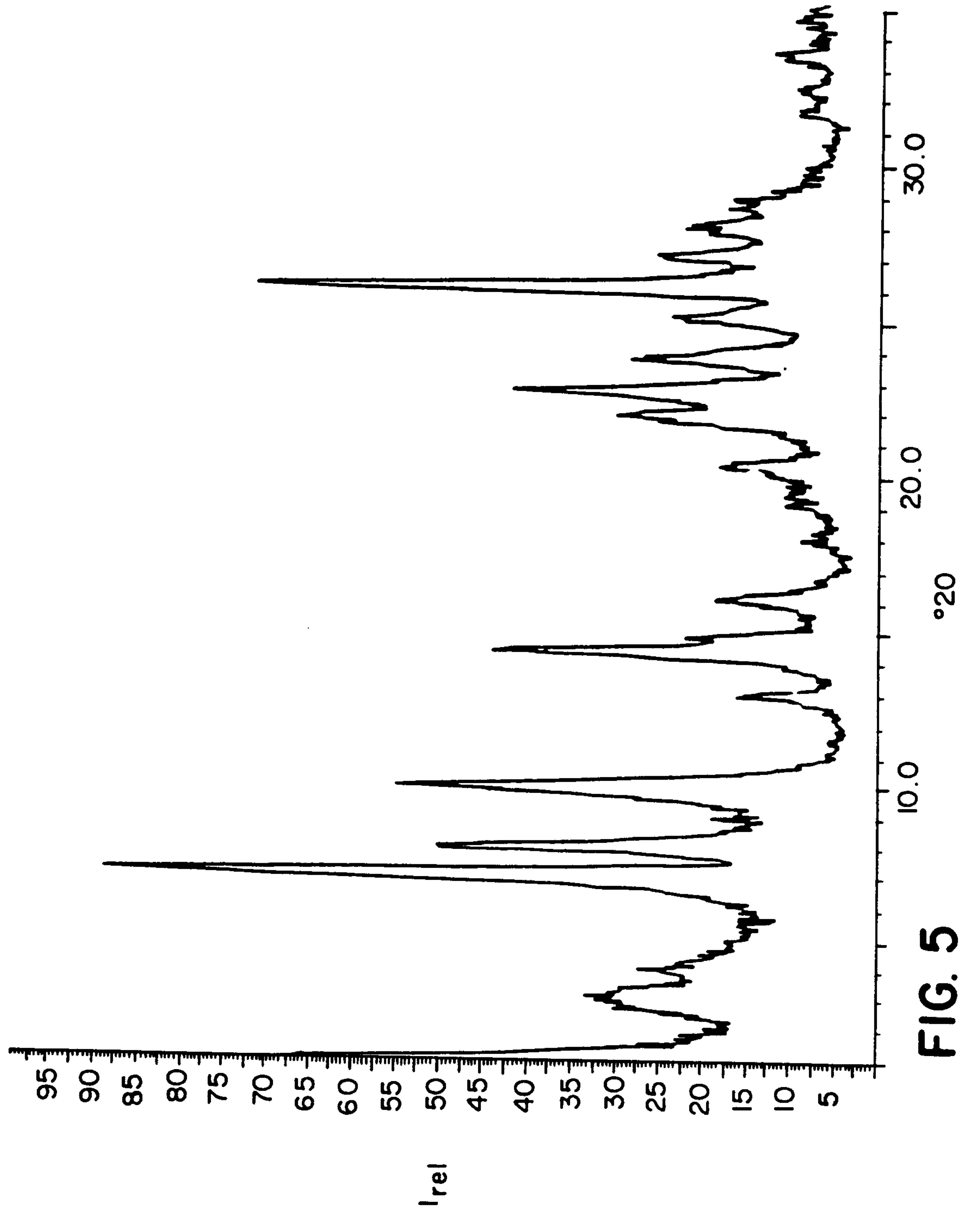
I rel
95
90
85
80
75
70
65
60
55
50
45
40
35
30
25
20
15
10
5
10.0
°2θ
20.0
30.0

FIG. 5